# EUROPEAN PATENT SPECIFICATION

(11) **EP 1 769 720 B1**
(45) Date of publication and mention of the grant of the patent: **15.06.2011**
(21) Application number: 05765470.9
(22) Date of filing: 05.07.2005
(51) Int. Cl.: A61B 1/00

(54) **DEVICE INTRODUCABLE INTO A SUBJECT**
IN EINE PERSON EINFÜHRBARE VORRICHTUNG
DISPOSITIF INTRODUISABLE DANS LE SUJET

(30) Priority: 08.07.2004 JP 2004201931
(43) Date of publication of application: 04.04.2007
(73) Proprietor: Olympus Corporation, Tokyo 151-0072 (JP)
(72) Inventor: SUZUSHIMA, Hiroshi, 3994601 (JP); FUJIMORI, Noriyuki, 3920131 (JP); ORIHARA, Tatsuya, 1920907 (JP); HOMAN, Masatoshi, 1910062 (JP); HONDA, Takemitsu, 1910062 (JP); NAKATSUCHI, Kazutaka, 1910062 (JP)
(74) Representative: von Hellfeld, Axel
(86) International application number: PCT/JP2005/012419
(87) International publication number: WO 2006/006452

(56) References cited:
- WO-A-02/102224
- JP-A- 2001 231 744
- JP-A- 2003 070 728
- JP-A- 2003 070 728
- JP-A- 2004 121 843
- US-A1- 2003 208 107

## Description

### TECHNICAL FIELD

The present invention relates to a body insertable apparatus, which is inserted into a subject and picks up an image inside the subject, and to a body insertable apparatus system.

### BACKGROUND ART

Recently, a swallowable capsule endoscope has been proposed in a field of endoscopes. The capsule endoscope has an imaging function and a radio transmission function. After being swallowed by a patient, i.e., a subject, from the mouth for an observation (examination), the capsule endoscope travels through inside body cavities, e.g. internal organs such as stomach and small intestine following peristaltic movements and sequentially captures images using the imaging function, according to which intra-subject images are captured at 0.5-second intervals, for example, until naturally discharged from a living body (human body) of the subject.

While the capsule endoscope travels through the internal organs, the capsule endoscope captures images in the body cavities thereby obtaining image data, and sequentially transmits the image data to an outside using the radio communication function. The image data is accumulated in a memory provided outside. When the subject carries the receiving apparatus equipped with the radio communication function and the memory function, the subject can move freely without inconveniences even after swallowing the capsule endoscope and before discharging the same. After the capsule endoscope is discharged, a doctor or a nurse can display images of the organs on a display unit or the like based on the image data accumulated in the memory, and make diagnosis (see, for example, Patent Document 1).

Generally, an imaging mechanism provided in the capsule endoscope has an optical system for focusing light supplied from outside, and a photoelectric transducer that converts the light focused by the optical system to electric signals. The capsule endoscope has a data generator that generates image data based on the electric signals output from the imaging mechanism. A necessary processing such as modulation is performed on the image data generated by the data generator, and the processed image data is radio transmitted to outside.

The aforementioned capsule endoscope having a plurality of imaging mechanisms is proposed. The plurality of imaging mechanisms are provided inside the capsule endoscope to obtain a plurality of image data, in which each image data corresponds to different field of view. Thus, obtainable information on the body cavity of a patient increases so that the doctor can make diagnosis on the body cavity more accurately.

Patent Document 1: Japanese Patent Application Laid-open No. 2003-19111

US 2003/0208107 A1 relates to an encapsulated medical imaging device and a method for imaging the gastrointestinal tract in patients using optical scanning technologies. For this purpose, an encapsulated medical imaging system is provided, comprising a capsule of swallowable proportion, comprising at least one optical setup of a number of optical setups comprising an array of microlenses distributed in axial symmetry on at least a portion of the capsule so that the array of microlenses is capable of receiving light reflected from object located in at least a sector outside the capsule, corresponding optical array comprising an array of light sensitive cells optically communicating with said optical setup through focusing means, such that the image of the object is focused on the array of light sensitive cells, electronic circuitry adapted to sample image data obtained by the optical array by scanning the image and converting the image data to digital data, illuminating means for illuminating a sector in front the optical setup, outside the capsule, transmitting means communicating with said electronic circuitry, adapted to receive image digital data and transmit it to an external receiver, receiving means for receiving data transmitted from said capsule, image processing means for processing the data received by the receiving means, and display means for displaying an image.

JP 2003070728 refers to a capsule type endoscope, comprising an imaging element, a lighting element, a battery, an image signal processing circuit, a memory, an image information transmission circuit, and a radio transmission antenna, which are housed in the capsule main body. Signals imaged by the imaging element are processed by the image signal processing circuit to generate image information. The generated image information is once stored in the memory and then transmitted to the outside of the body by radio by the image information transmission circuit.

### DISCLOSURE OF INVENTION

### PROBLEM TO BE SOLVED BY THE INVENTION

However, when the capsule endoscope comes to have the plurality of imaging mechanisms, a size of the capsule endoscope increases due to an increase in a number of elements provided therein, and a number of wirings electrically connecting the elements increases correspondingly. Normally, each element housed in the capsule endoscope is arranged on a different board. Hence, as the number of elements increases, the number of boards housed in the capsule endoscope increases, and inside the capsule endoscope, a region occupied by the boards increases. Further, it is required to electrically connect the elements with each other. Hence, the number of the wirings electrically connecting the boards increases along with the increase in the number of the boards. As a result, the size of the capsule endoscope increases, and probability of disconnection of the wirings increases along with the increase in the number of the wirings. Therefore, the capsule endoscope having the aforementioned configuration is not appropriate.

The present invention is provided in view of the foregoing, and an object of the present invention is to realize a body insertable apparatus, which suppresses increase in the number of boards due to increase in the number of elements, and to realize a body insertable apparatus system.

### MEANS FOR SOLVING PROBLEM

A body insertable apparatus according to the present invention is defined by the independent claim. Preferred embodiments of the body insertable apparatus are defined by the dependent claims.

### EFFECT OF THE INVENTION

In a body insertable apparatus according to the present invention, a first photoelectric transducer and a second photoelectric transducer are arranged on a same face of the surface of the imaging board. Consequently, a number of boards provided inside an outer casing member can be reduced, and increase in size of the body insertable apparatus can be suppressed.

### BRIEF DESCRIPTION OF DRAWINGS

FIG. 1 is a general schematic diagram of a body insertable apparatus system according to a first embodiment;
FIG. 2 is a block diagram of a receiving device provided in the body insertable apparatus system;
FIG. 3 is a schematic diagram of a configuration of an capsule endoscope provided in the body insertable apparatus system;
FIG. 4 is a block diagram for explaining connection relationships among elements provided in the capsule endoscope;
FIG. 5 is a schematic diagram of an illuminating board provided in an capsule endoscope according to a modification; and
FIG. 6 is a schematic diagram of a configuration of an capsule endoscope provided in a body insertable apparatus system according to a second embodiment.

### EXPLANATIONS OF LETTERS OR NUMERALS

- 1: Subject
- 2: Capsule endoscope
- 3: Receiving device
- 4: Display device
- 5: Portable recording medium
- 6a-6h: Receiving antennas
- 9: Antenna selector
- 10: Receiving circuit
- 11: Signal processing unit
- 12: Control unit
- 13: Storage unit
- 14: A/D converter
- 15: Power supply unit
- 17: Outer casing member
- 17a: Imaging window
- 17b: Imaging window
- 18: Imaging board
- 19: First imaging mechanism
- 19a: First imaging device
- 19b: First optical system
- 19c: Holder member
- 20: Second imaging mechanism
- 20a: Second imaging device
- 20b: Second optical system
- 20c: Holder member
- 21: First illuminating board
- 22: First illuminating unit
- 23: Second illuminating board
- 24: Second illuminating unit
- 25: Transmitting unit
- 25a: Transmitting board
- 25b: transmitting antenna
- 26: Power unit
- 26a: Power board
- 26b,: 26c Storage battery
- 27: Data generator
- 28: Timing controller
- 29: Wiring configuration
- 29a: Printed wiring
- 29b: Through hole
- 30: Selector
- 32: First illuminating board
- 33: Second illuminating board
- 34, 35: Opening
- 37: Capsule endoscope
- 38: Outer casing member
- 38a, 38b: Imaging window
- 39a, 39b: Bent portion
- 39: Imaging board
- 40: Transmitting unit

### BEST MODE(S) FOR CARRYING OUT THE INVENTION

Hereinafter, embodiments of a body insertable apparatus and a body insertable apparatus system according to the present invention is explained. It should be noted that the accompanying drawings are merely schematic, and relation between width and thickness of each portion, thickness ratio of one portion to another, and the like may be different in an actual apparatus and a system. The dimensional relations and the ratio may be different from one drawing to another.

### First Embodiment

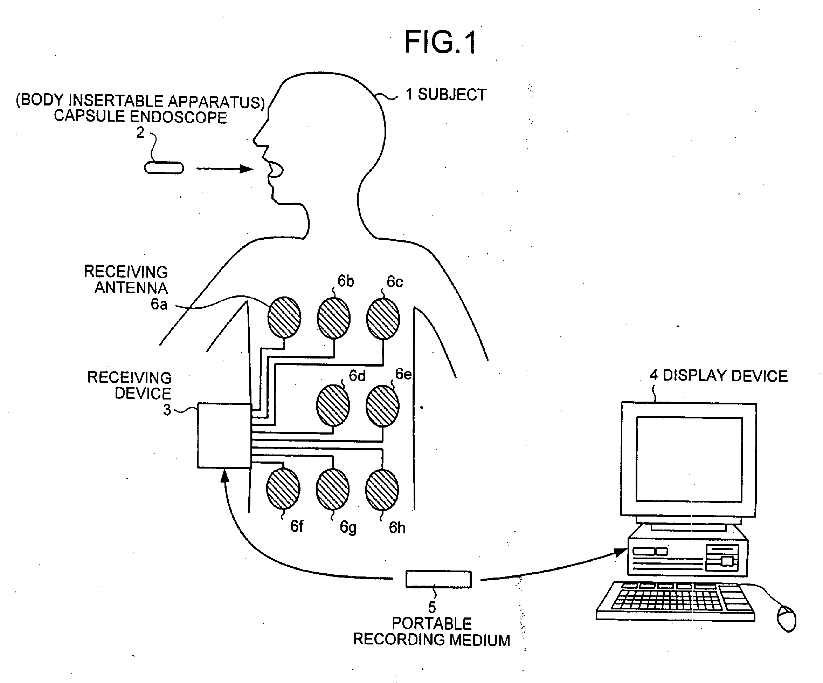

A body insertable apparatus system according to a first embodiment is explained. FIG. 1 is a general schematic diagram of the body insertable apparatus system according to the first embodiment. As shown in FIG. 1, the body insertable apparatus system according to the first embodiment has an capsule endoscope 2, a receiving device 3, a display device 4, and a portable recording medium 5. The capsule endoscope 2 is inserted into a subject 1, and travels along a traveling passage. The receiving device 3 receives radio signals, which are transmitted from the capsule endoscope 2 and contain subject interior information. The display device 4 displays content on the subject interior information contained in the radio signals received by the receiving device 3. The portable recording medium 5 transfers information between the receiving device 3 and the display device 4.

The display device 4 serves to display a subject interior image and the like picked up by the capsule endoscope 2 and received by the receiving device 3, and the display device 4 has a configuration such as a workstation that displays the image based on data acquired from the portable recording medium 5. Specifically, the display device 4 may have a configuration that directly displays the image through a cathode ray tube (CRT) display, a liquid crystal display, and the like, or may have a configuration that outputs the image to other medium, such as a printer.

The portable recording medium 5 is detachable with respect to the receiving device 3 and the display device 4, and can record or output the information when the portable recording medium 5 is attached to the receiving device 3 or the display device 4. Specifically, the portable recording medium 5 is attached to the receiving device 3 and records the subject interior image, while the capsule endoscope 2 travels through inside the body cavity of the subject 1. After the capsule endoscope 2 is discharged from the subject 1, the portable recording medium 5 is removed from the receiving device 3, and attached to the display device 4. Then, the display device 4 reads the data recorded on the portable recording medium 5. Unlike when the receiving device 3 is connected to the display device 4 through a cable, the subject 1 can freely move while the capsule endoscope 2 travels through inside the subject 1, since the data is transferred between the receiving device 3 and the display device 4 through a portable recording medium 5 consisting of a compact flash® memory and the like.

Receiving antennas 6a to 6h consist of, for example, loop antennas. During their use, the loop antennas are fixed on predetermined positions of a body surface of the subject 1, and the receiving antennas 6a to 6h preferably have securing units for fixing the loop antennas on the body surface of the subject 1.

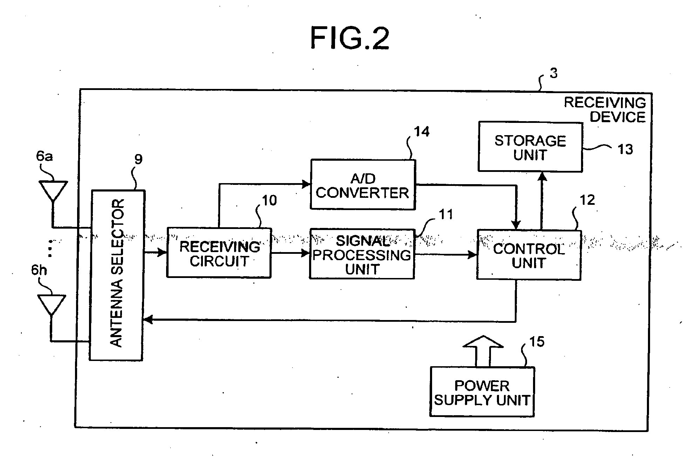

The receiving device 3 serves to perform a receiving processing on the radio signals received through one of the receiving antennas 6a to 6h. FIG. 2 is a block diagram of the receiving device 3. As shown in FIG. 2, the receiving device 3 has an antenna selector 9, a receiving circuit 10, and a signal processing unit 11. The antenna selector 9 selects a receiving antenna, which is appropriate for receiving the radio signals, from the receiving antennas 6a to 6h. The receiving circuit 10 performs a processing such as demodulation on the radio signals received through receiving antenna 6 selected by the antenna selector 9. The signal processing unit 11 extracts the subject interior image, information on detected magnetic field, and the like from the radio signals after the processing. Further, the receiving device 3 has a control unit 12, a storage unit 13, an A/D (analog/digital) converter 14, and a power supply unit 15. The control unit 12 controls the output and the like of the extracted information in a predetermined manner. The storage unit 13 stores the extracted information. The A/D converter 14 performs an A/D conversion on analog signals supplied from the receiving circuit 10 and corresponding to strength of the received radio signals. The power supply unit 15 supplies driving power of the aforementioned elements provided in the receiving device 3.

The antenna selector 9 serves to select the antenna, which is appropriate for receiving the radio signals, from the receiving antennas 6a to 6h. Specifically, the antenna selector 9 selects the predetermined receiving antenna 6 under the control of the control unit 12, and outputs the radio signals received through the selected receiving antenna 6 to the receiving circuit 10.

The receiving circuit 10 serves to perform the predetermined processing such as the demodulation on the radio signals received through the selected receiving antenna 6. The receiving circuit 10 outputs the analog signals, which correspond to the strength of the radio signals, to the A/D converter 14.

The signal processing unit 11 serves to extract predetermined information from the signals, on which the predetermined processing is performed by the receiving circuit 10. For example, when the radio signals to be received by the receiving device 3 are transmitted from an electronic device having an imaging function, the signal processing unit 11 extracts the image data from the signals output from the receiving circuit 10.

The control unit 12 serves to perform a general controlling of operations including an antenna selection operation performed by the antenna selector 9.
Specifically, the control unit 12 transfers the information output from the signal processing unit 11 to the storage unit 13, and stores the transferred information in the storage unit 13. Further, the control unit 12 selects the receiving antenna 6 to be used based on digital signals (for example, received signal strength indicator (RSSI)), which are output from the A/D converter 14, corresponding to the receiving strength, and the control unit 12 commands the antenna selector 9 to select the receiving antenna 6.

The storage unit 13 serves to store the information extracted by the signal processing unit 11. Specifically, the storage unit 13 may have a memory and the like that store the information; however, in the first embodiment, the storage unit 13 writes the information into the portable recording medium 5.

The capsule endoscope 2 is explained. The capsule endoscope 2 functions as a body insertable apparatus. The capsule endoscope 2 acquires the image data inside the subject 1, and transmits the radio signals containing the acquired image data to the receiving device 3.

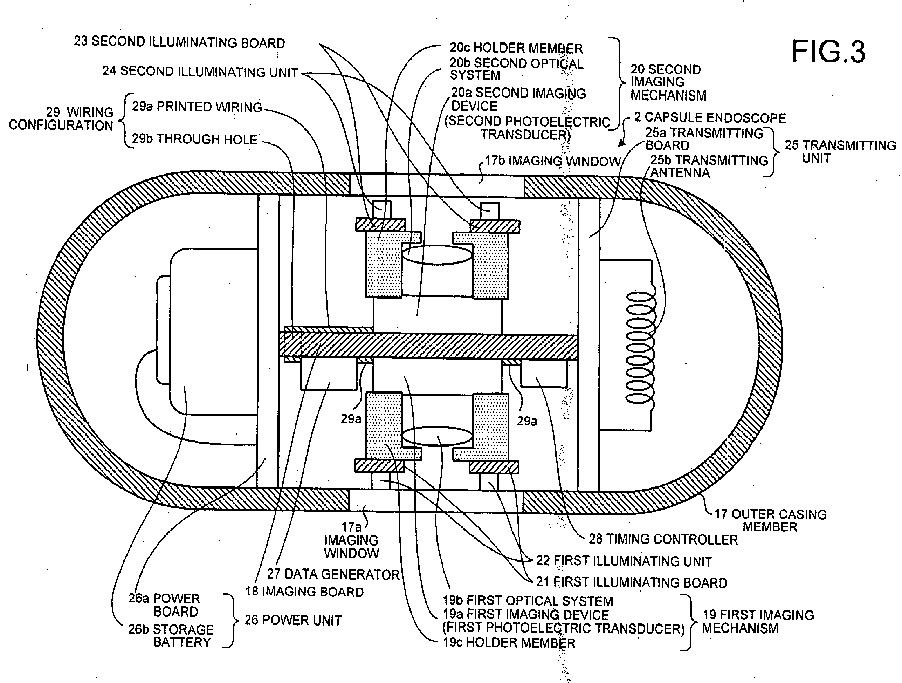

FIG. 3 is a sectional view of a specific configuration of the capsule endoscope 2. As shown in FIG. 3, the capsule endoscope 2 has an imaging board 18, a first imaging mechanism 19, a second imaging mechanism 20, a first illuminating unit 22, and a second illuminating unit 24. The imaging board 18 is secured at a predetermined position inside an outer casing member 17 determining an outer shape of the capsule endoscope 2. The first imaging mechanism 19 is arranged on one face (first face) of the imaging board 18. The second imaging mechanism 20 is arranged on other face (second face) of the imaging board 18. The first illuminating unit 22 is arranged on a first illuminating board 21 arranged near the first imaging mechanism 19. The second illuminating unit 24 is arranged on a second illuminating board 23 arranged near the second imaging mechanism 20. Further, the capsule endoscope 2 has a data generator 27, a timing controller 28, and a wiring configuration 29, and each of the data generator 27, the timing controller 28, and the wiring configuration 29 is arranged on the imaging board 18. The data generator 27 generates image data based on electric signals obtained by the first imaging mechanism 19 and the second imaging mechanism 20. The timing controller 28 controls driving timings and the like of at least the first imaging mechanism 19, the second imaging mechanism 20, and the data generator 27. The wiring configuration 29 electrically connects the aforementioned elements inside the capsule endoscope 2. Further, the capsule endoscope 2 has a transmitting unit 25 that transmits the radio signals containing the image data obtained by the data generator 27, and a power unit 26 that supplies driving power to the first imaging mechanism 19 and the like through the wiring configuration 29.

The transmitting unit 25 serves to transmit the radio signals to the receiving device 3. Specifically, the transmitting unit 25 is arranged at a predetermined position inside the outer casing member 17, and has a transmitting board 25a, on which electronic circuit required to perform modulation and the like is formed, and a transmitting antenna 25b that transmits signals on which the processing is performed by the electronic circuit formed on the transmitting board 25a.

The power unit 26 serves to supply the driving power to the elements, such as the first imaging mechanism 19, provided inside the capsule endoscope 2. Specifically, the power unit 26 has a power board 26a on which a required electronic circuit including electrodes is formed, and a storage battery 26b arranged on the power board 26a and electrically connected to the electrodes formed on the power board 26a.

The imaging board 18 serves to support the elements such as the first imaging mechanism 19 and the second imaging mechanism 20. Specifically, the first imaging mechanism 19 is arranged on the first face of the imaging board 18, the second imaging mechanism 20 is arranged on the second face opposing to the first face, and the data generator 27 and the timing controller 28 are arranged on one of the first face and the second face. Since the elements are arranged on the same board, the wiring configuration 29 that electrically connects the elements is formed on the imaging board 18. The wiring configuration 29 includes a through hole 29b that electrically connects the first face and the second face of the imaging board 18 to each other, in addition to a printed wiring configuration 29a formed on a surface of the imaging board 18.

The first imaging mechanism 19 serves to convert external light coming through an imaging window 17a formed at the outer casing member 17 to electric signals. Specifically, the first imaging mechanism 19 has a first imaging element 19a that functions as a photoelectric transducer, a first optical system 19b that focuses the external light coming through the imaging widow 17a on a light receiving face of the first imaging element 19a, and a holder member 19c that secures the first optical system 19b therein.

The first imaging element 19a outputs electric signals corresponding to strength of light focused on the predetermined light receiving face, and functions as a first photoelectric transducer. Specifically, the first imaging element 19a consists of a charge coupled device (CCD), and has a photoelectric transducer such as a photodiode arranged in a matrix shape on the predetermined light receiving face. In the first embodiment, the first imaging element 19a has a predetermined electrical connecting terminal (not shown) at a section that is in contact with the first face of the imaging board 18, and the first imaging element 19a is electrically connected to the wiring configuration 29 formed on the imaging board 18 through the connecting terminal.

The first optical system 19b serves to focus the external light coming through the imaging window 17a on the light receiving face of the first imaging element 19a. In the example of FIG. 3, the first optical system 19b consists of a single lens; however, the present invention is not limited thereto. Hence, the first optical system 19b may consist of a combination of a plurality of lenses, or may have other mechanism having the focusing function.

The second imaging mechanism 20 serves to convert the external light coming through an imaging window 17b formed at the outer casing member 17 to electric signals. Specifically, as similar to the first imaging mechanism 19, the second imaging mechanism 20 has a second imaging element 20a, a second optical system 20b, and a holder member 20c that secures the second optical system 20b therein. As similar to the first imaging device 19a, the second imaging device 20a consists of a CCD and the like, and has a predetermined connecting terminal at a section that is in contact with the second face of the imaging board 18. The second optical system 20b has a configuration similar to the configuration of the first optical system 19b, and the holder member 20c has a configuration that is similar to the configuration of the holder member 19c; therefore, explanations thereof are not to be repeated.

The first illuminating unit 22 and the second illuminating unit 24 serve to output illuminating light that illuminates tissue, i.e., an imaging object, inside the subject during the imaging operation by the first imaging mechanism 19 and the second imaging mechanism 20. Specifically, the first illuminating unit 22 and the second illuminating unit 24 consist of a light emitting diode (LED) and the like, and the first illuminating unit 22 and the second illuminating unit 24 output the illuminating light with timings in synchronization with the imaging operation of the first imaging mechanism 19 and the second imaging mechanism 20, respectively.

The timing controller 28 serves to at least control operation timings of the elements, such as the first imaging mechanism 19, arranged on the imaging board 18. Specifically, the timing controller 28 includes, for example, a timing generator that generates pulse signals, which is reference to the driving timing, and the timing controller 28 outputs controlling signals generated based on the reference pulse signals to each element.

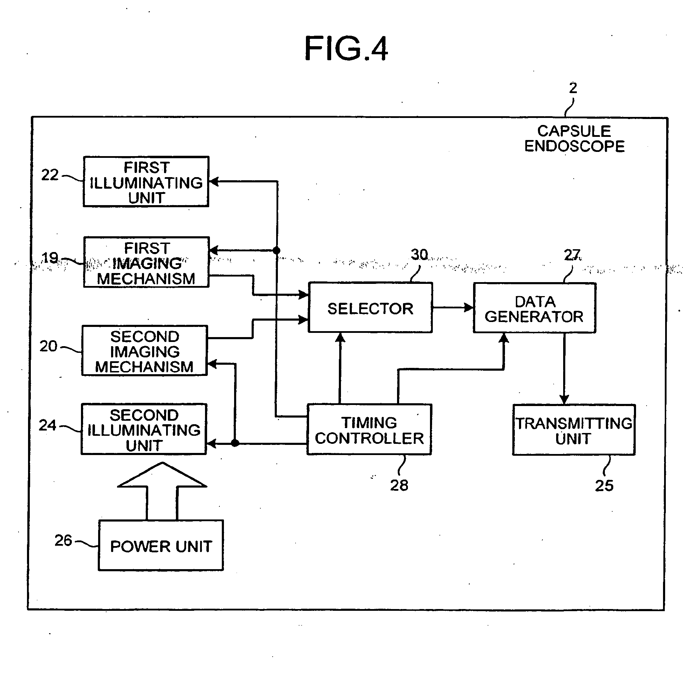

Connection relationships among the elements provided in the capsule endoscope 2 are explained. FIG. 4 is a block diagram of the connection relationships among the elements provided in the capsule endoscope 2. As shown in FIG. 4, in the capsule endoscope 2, the power unit 26 supplies the driving power to each element, and the timing controller 28 Controls the driving timings of the first imaging mechanism 19, the second imaging mechanism 20, the first illuminating unit 22, the second illuminating unit 24, a selector 30 (described later), and the data generator 27. The electric signals acquired by one of the first imaging mechanism 19 and the second imaging mechanism 20 are selected when the electric signals passes through the selector 30, and the selected electric signals are output to the data generator 27 to generate the image data. The image data generated by the data generator 27 is output to the transmitting unit 25, and the modulation and the like is performed on the output image data, if necessary. Then, the image data is output to the receiving device 3.

The selector 30 serves to select the electric signals output from one of the first imaging mechanism 19 and the second imaging mechanism 20, and outputs the selected electric signals to the data generator 27. Although the selector 30 is not shown in FIG. 3, the selector 30 is also arranged on the imaging board 18. In FIG. 4, the data generator 27 and the selector 30 are provided separately from one other; however, for example, a data generator having a data selecting function can replace the data generator 27 and the selector 30.

In the connection relationships among the aforementioned elements, the elements used for an image data generation, i.e., the first imaging mechanism 19, the second imaging mechanism 20, the first illuminating unit 22, the second illuminating unit 24, the selector 30, the data generator 27, and the timing controller 28, are connected to each other by the wiring configuration 29 formed on the imaging board 18. Hence, among the connections shown in FIG. 4, the connections excluding the connection of an output wiring transferring the generated image data to the transmitting unit 25 are formed by the wiring configuration on the imaging board 18.

Advantages associated with the body insertable apparatus system according to the first embodiment is explained. In the aforementioned capsule endoscope 2 of the first embodiment, the first imaging mechanism 19 and the second imaging mechanism 20 are arranged on the single imaging board 18. Thus, a number of boards provided in the capsule endoscope 2 can be reduced compared to when the first imaging mechanism 19 and the second imaging mechanism 20 are each arranged on a different board.

In the first embodiment, the first imaging mechanism 19 is arranged on the first face of the imaging board 18, and the second imaging mechanism 20 is arranged on the second face that is different from the first face. Since the first embodiment employs the aforementioned configuration, an imaging field of view of the first imaging mechanism 19 differs from an imaging field of view of the second imaging mechanism 20, and a subject interior image associated with a wider range can be obtained.

The first imaging mechanism 19 and the second imaging mechanism 20, and in addition, the elements, such as the data generator 27, associated with the generation of the image data are each arranged on the imaging board 18. Since the wiring configuration 29 formed on the imaging board 18 electrically connects each element, a region occupied by the wiring configuration provided inside the capsule endoscope 2 can be reduced. Particularly, when the plurality of imaging mechanisms are provided as similar to the first embodiment, the number of the wiring configuration running towards the data generator 27 from the imaging mechanisms increases by the increased number of the imaging mechanisms. When the data generator 27 is arranged on a board that is different from a board on which the imaging mechanisms are arranged, the number of the wiring configuration used to connect the boards is increased compared to when a single imaging mechanism is provided. Thus, the region occupied by the wiring configuration increases. In the first embodiment, however, the first imaging mechanism 19, the second imaging mechanism 20, the data generator 27 can be connected to each other by printed wiring 29a and the like. Thus, even when the number of the imaging mechanisms is increased, the region occupied by the wiring configuration inside an interior space region of the capsule endoscope 2 does not increase, and it can be prevented to increase a size of the capsule endoscope 2.

### Modification

A modification of a body insertable apparatus system according to the first embodiment is explained. In the modification, a first illuminating board and a second illuminating board provided in the capsule endoscope are curved so as to match with a shape of an inner face of the outer casing member of the capsule endoscope.

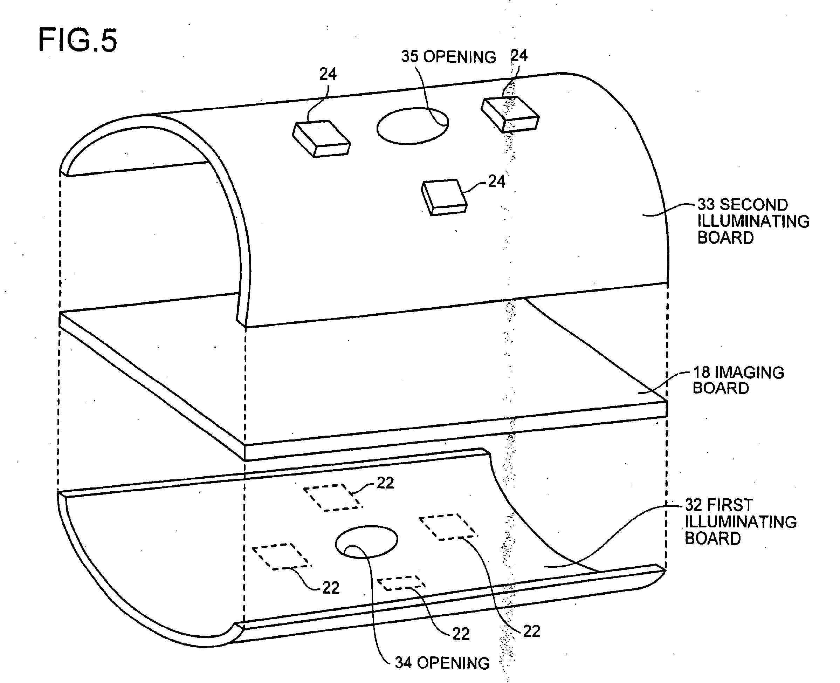

FIG. 5 is a schematic diagram of the first illuminating board and the second illuminating board according to the modification. As shown in FIG. 5, a first illuminating board 32 and a second illuminating board 33 provided in the capsule endoscope are each curved so as to match with the shape of the inner face of the outer casing member 17, and has the first illuminating board 32 and the second illuminating board 33 having openings 34 and 35, respectively, so that the light from outside enters the first imaging mechanism 19 and the second imaging mechanism 20. The first illuminating board 32 and the second illuminating board 33 are arranged so as to substantially contact with the inner face of the outer casing member 17.

Generally, the outer casing member 17 of the capsule endoscope has a shape in which semispherical dorm members are fixed on both ends of a cylindrical member , and it is apparent from FIG. 3 that the first illuminating unit 22 and the second illuminating unit 24 are arranged inside the cylindrical member. In the modification, shapes of the first illuminating board 32 and the second illuminating board 33 having the first illuminating unit 22 and the second illuminating unit 24, respectively, have semicylindrical shapes so as to match with the shape of the inner face of the outer casing member 17. To realize the aforementioned shape, the first illuminating board 32 and the second illuminating board 33 can be formed with flexible boards having flexibility.

By the capsule endoscope employing the aforementioned configuration, a region, in which the elements other than the first illuminating board 32 and the like are arranged, can sufficiently be obtained. Since the first illuminating board 32 and the second illuminating board 33 have the curved shapes that match with the shape of the inner face of the outer casing member 17, spaces between the outer casing member 17 and each of the first illuminating board 32 and the second illuminating board 33 can be reduced, and the region in which other elements are arranged can sufficiently be obtained on the inner face (a face opposite to a face in front of the outer casing member 17) of the first illuminating board 32 and the like.

Depressed portions housing the first illuminating unit 22 and the second illuminating unit 24 can be formed on, for example, the first illuminating board 32 and the second illuminating board 33. By the capsule endoscope employing the aforementioned configuration, height of protrusions formed by the first illuminating unit 22 and the like can be reduced or eliminated. Thus, a space region between the board such as the first illuminating board 32 and the outer casing member 17 can be reduced.

### Second Embodiment

A body insertable apparatus system according to a second embodiment is explained. In the body insertable apparatus system according to the second embodiment, an imaging board provided in the capsule endoscope has a predetermined bent portion, and the bent portion forms a U-shaped cross section of the imaging board.

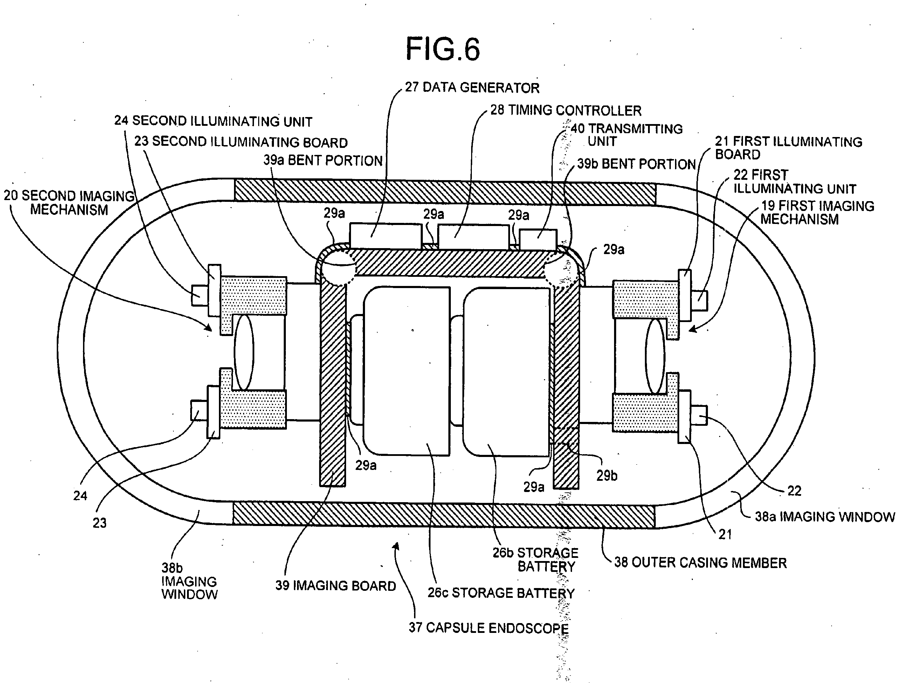

FIG. 6 is a sectional view of a capsule endoscope 37 provided in the body insertable apparatus system according to the second embodiment. Even though not shown, the body insertable apparatus system according to the second embodiment has the receiving device 3, the display device 4, the portable recording medium 5, and the receiving antennas 6a to 6h, as similar to the first embodiment. Among the elements shown in FIG. 6, elements represented by names, letters, and numerals that are similar to those of the first embodiment have configurations and functions similar to those of the first embodiment as long as not specifically mentioned hereinafter.

As shown in FIG. 6, the capsule endoscope 37 has an imaging board 39 that is bent at bent portions 39a and 39b and formed in the U-shape, in an outer casing member 38 that determines an exterior shape of the capsule endoscope 37. In the imaging board 39, the first imaging mechanism 19, the second imaging mechanism 20, the data generator 27, the timing controller 28, and a transmitting unit 40 are arranged on a face (outer face) of a protruding region side formed by the U-shape. Further, in the imaging board 39, batteries 26b and 26c (corresponding to a power supply unit) connected in series are arranged in a space region, which is formed by a face (inner face) of a depressed portion side formed by the U-shape, so that a cathode of one battery and an anode of another battery are in contact with the electrodes, respectively, formed on the face of the depressed portion side of the imaging board 39.

The first imaging mechanism 19 and the second imaging mechanism 20 are arranged so that optical axes of the optical system match with a traveling direction and a direction opposite to the traveling direction (i.e., longitudinal direction of the outer casing member 38) of the capsule endoscope 37. Imaging windows 38a and 38b are formed corresponding to imaging field of views of the first imaging mechanism 19 and the second imaging mechanism 20, respectively, on the outer casing member 38. Electrical properties of each of the first imaging mechanism 19, the second imaging mechanism 20, the data generator 27, and the timing controller 28 are the same as the electrical properties explained in the first embodiment, and as similar to the first embodiment, elements are electrically connected to each other by the wiring configuration 29 such as the printed wiring configuration 29a and the through hole 29b. To realize the configuration having the bent portion, it is preferred to form the imaging board 39 by a flexible board or a rigid/flexible composite board, or at least regions corresponding to the bent portions 39a and 39b are preferred to be made by a flexible board that can easily be bent.

Advantages associated with the body insertable apparatus system according to the second embodiment is explained. As similar to the first embodiment, in the body insertable apparatus system according to the second embodiment, both the first imaging mechanism 19 and the second imaging mechanism 20 are arranged on the single imaging board 39, so that an advantage such that the number of the boards can be reduced is obtained.

In the body insertable apparatus system according to the second embodiment, the imaging board 39 provided in the capsule endoscope 37 has the bent portions 39a and 39b so that the cross section of the imaging board 39 has U-shape. Therefore, even when the imaging field of views of the first imaging mechanism 19 and the second imaging mechanism 20 extend in the longitudinal direction of the outer casing member 38, the first imaging mechanism 19 and the second imaging mechanism 20 may be arranged on the same imaging board 39. As similar to the first embodiment, when the first imaging mechanism 19 and the second imaging mechanism 20 are arranged on the front face and the back face, respectively, of the plate-like board having no bent portion, other elements such as the batteries 26b and 26c are required to be arranged, with respect to the first imaging mechanism 19 and the like, in a short side direction of the outer casing member 38 to avoid blocking the imaging field of views. Consequently, a size of the capsule endoscope 37 increases. On the other hand, in the body insertable apparatus system according to the second embodiment, the cross section of the imaging board 39 provided in the capsule endoscope 37 has the U-shape, and the batteries 26b and 26c are arranged on the depressed region side of the U-shape. Therefore, even though the imaging field of views of the first imaging mechanism 19 and the second imaging mechanism 20 extend in the longitudinal direction of the outer casing member 38, the increase in the size of the capsule endoscope 37 can be prevented.

### INDUSTRIAL APPLICABILITY

As described hereinbefore, a body insertable apparatus and a body insertable apparatus system according to the present invention are useful for an image capturing process of an image of a subject interior such as a body cavity interior, and appropriate for a body insertable apparatus and a body insertable apparatus system that can suppress increase in size of the body insertable apparatus while having an imaging function for obtaining a plurality of images, each corresponding to a different field of view inside the subject.

## Claims

1. A body insertable apparatus (2) which is insertable into a subject (1) and is adapted to pick up an image inside the subject (1), comprising:
an outer casing member (38) that determines an outer shape of the body insertable apparatus (2);
an imaging board (39) that is arranged inside the outer casing member (38);
a first imaging mechanism (19) that includes a first optical system (19b), the viewing direction of which matching with a travelling direction of the body insertable apparatus (2), and a first photoelectric transducer (19a) that is adapted to photoelectrically convert light coming through the first optical system (19b); and
a second imaging mechanism (20) that includes a second optical system (20b), the viewing direction of which being opposite to the viewing direction of the first optical system (19b), and a second photoelectric transducer (20a) that is adapted to photoelectrically convert light coming through the second optical system (20b);
wherein the first imaging mechanism (19) and the second imaging mechanism (20) are arranged on different regions of the same face of the surface of the imaging board (39),
the optical axis of the first optical system and the optical axis of the second optical system match an axis defining the travelling direction of the body insertable apparatus,
the imaging board (39) is bent at two bent portions to form a U-shaped cross section and arranged inside the outer casing member (38), so that the face of the surface of the imaging board (39) on which the first imaging mechanism (19) and the second imaging mechanism (20) are arranged is a face of a protruding region side of the U-shaped imaging board (39), and
the body insertable apparatus further comprises batteries (26b, 26c) connected in series and arranged in a space region, which is formed by an inner face of a depressed portion side formed by the U-shape, so that a cathode of one battery and an anode of another battery are in contact with the electrodes, respectively, formed on the inner face of the depressed portion side of the imaging board (39).

2. The body insertable apparatus (2) according to claim 1, further comprising:
a data generator (27) that is arranged on a face of the surface of the imaging board (39), the face of the surface being the face of the surface on which the first imaging mechanism (19) and the second imaging mechanism (20) are arranged, and is adapted to generate image data based on electric signals output from the first photoelectric transducer (19a) and the second photoelectric transducer (20a).

3. The body insertable apparatus (2) according to claim 2, wherein
the first photoelectric transducer (19a) and the data generator (27) are electrically connected to each other through a wiring configuration (29a) formed on the face of the surface of the imaging board (39) on which the first imaging mechanism (19) and the second imaging mechanism (20) are arranged, and
the second photoelectric transducer (20a) and the data generator (27) are electrically connected to each other through a wiring configuration (29a) formed on the face of the surface of the imaging board (39) on which the first imaging mechanism (19) and the second imaging mechanism (20) are arranged.

4. The body insertable apparatus (2) according to claim 2, further comprising
a transmitting unit (40) that is arranged on the face of the surface of the imaging board (39) on which the first imaging mechanism (19) and the second imaging mechanism (20) are arranged, and is adapted to transmit radio signals including the image data generated by the data generator (27).

## Patentansprüche

1. In einen Körper einführbare Vorrichtung (2), die in einen Patienten (1) einführbar ist und ein Bild in dem Patienten (1) aufzunehmen vermag, mit:
einem äußeren Gehäuseelement (38), das eine äußere Form der in einen Körper einführbaren Vorrichtung (2) festlegt;
einer Bildgebungsplatte (39), die innerhalb des äußeren Gehäuseelements (38) angeordnet ist;
einem ersten Bildgebungsmechanismus (19), der ein optisches System (19b), dessen Betrachtungsrichtung mit einer Bewegungsrichtung der in einen Körper einführbaren Vorrichtung (2) übereinstimmt, und einen ersten photoelektrischen Wandler (19a) enthält, der durch das erste optische System (19b) kommendes Licht photoelektrisch umzuwandeln vermag; und
einem zweiten Bildgebungsmechanismus (20), der ein zweites optisches System (20b), dessen Betrachtungsrichtung entgegengesetzt zu der Betrachtungsrichtung des ersten optischen Systems (19b) ist, und einen zweiten photoelektrischen Wandler (20a) enthält, der durch das zweite optische System (20b) kommendes Licht photoelektrisch umzuwandeln vermag;
wobei die erste Bildgebungsvorrichtung (19) und die zweite Bildgebungsvorrichtung (20) in verschiedenen Bereichen derselben Seite der Oberfläche der Bildgebungsplatte (39) angeordnet sind,
die optische Achse des ersten optischen Systems und die optische Achse des zweiten optischen Systems mit einer Achse übereinstimmen, welche die Bewegungsrichtung der in einen Körper einführbaren Vorrichtung festlegt,
die Bildgebungsplatte (39) an zwei Biegeabschnitten gebogen ist, um einen U-förmigen Querschnitt zu bilden, und in dem äußeren Gehäuseelement (38) so angeordnet ist, dass die Vorderseite der Oberfläche der Bildgebungsplatte (39), auf der der erste Bildgebungsmechanismus (19) und der zweite Bildgebungsmechanismus (20) angeordnet sind, eine Vorderseite einer Vorsprungsbereichsseite der U-förmigen Bildgebungsplatte (39) ist, und
die in einen Körper einführbare Vorrichtung ferner Batterien (26b, 26c) umfasst, die in Reihe geschaltet und in einem Raumbereich angeordnet sind, der durch eine Innenfläche einer durch die U-Form gebildeten Vertiefungsabschnittsseite gebildet ist, so dass eine Kathode einer Batterie und eine Anode einer anderen Batterie jeweils mit den an der Innenfläche der Vertiefungsabschnittsseite der Bildgebungsplatte (39) ausgebildeten Elektroden in Kontakt stehen.

2. In einen Körper einführbare Vorrichtung (2) nach Anspruch 1, ferner mit:
einem Datengenerator (27), der auf einer Vorderseite der Oberfläche der Bildgebungsplatte (39) angeordnet ist, wobei die Vorderseite der Oberfläche diejenige Vorderseite der Oberfläche ist, auf der der erste Bildgebungsmechanismus (19) und der zweite Bildgebungsmechanismus (20) angeordnet sind, und der Bilddaten auf der Basis von von dem ersten photoelektrischen Wandler (19a) und dem zweiten photoelektrischen Wandler (20a) ausgegebenen elektrischen Signalen zu erzeugen vermag.

3. In einen Körper einführbare Vorrichtung (2) nach Anspruch 2, wobei
der erste photoelektrische Wandler (19a) und der Datengenerator (27) über eine Verdrahtungskonfiguration (29a) miteinander verbunden sind, die an der Vorderseite der Oberfläche der Bildgebungsplatte (39), an der der erste Bildgebungsmechanismus (19) und der zweite Bildgebungsmechanismus (20) angeordnet sind, ausgebildet ist, und
der zweite photoelektrische Wandler (20a) und der Datengenerator (27) über eine Verdrahtungskonfiguration (29a) miteinander verbunden sind, die an der Vorderseite der Oberfläche der Bildgebungsplatte (39), an der der erste Bildgebungsmechanismus (19) und der zweite Bildgebungsmechanismus (20) angeordnet sind, ausgebildet ist.

4. In einen Körper einführbare Vorrichtung (2) nach Anspruch 2, ferner mit
einer Übertragungseinheit (40), die an der Vorderseite der Oberfläche der Bildgebungsplatte (39), an der der erste Bildgebungsmechanismus (19) und der zweite Bildgebungsmechanismus (20) angeordnet sind, angebracht ist und die Funksignale, welche die von dem Datengenerator (27) erzeugten Bilddaten enthalten, zu übertragen vermag.

## Revendications

1. Appareil (2) insérable dans un corps qui est insérable dans un sujet (1) et est adapté à capturer une image à l'intérieur du sujet (1), comprenant :
un élément de boîtier extérieur (38) qui détermine une forme extérieure de l'appareil (2) insérable dans un corps ;
une carte d'imagerie (39) qui est agencée à l'intérieur de l'élément de boîtier extérieur (38) ;
un premier mécanisme d'imagerie (19) qui inclut un premier système optique (19b), le sens de visée duquel correspondant avec un sens de déplacement de l'appareil (2) insérable dans un corps, et un premier transducteur photoélectrique (19a) qui est adapté à convertir photoélectriquement une lumière passant à travers le premier système optique (19b) ; et
un deuxième mécanisme d'imagerie (20) qui inclut un deuxième système optique (20b), le sens de visée duquel étant opposé au sens de visée du premier système optique (19b), et un deuxième transducteur photoélectrique (20a) qui est adapté à convertir photoélectriquement une lumière passant à travers le deuxième système optique (20b) ;
dans lequel le premier mécanisme d'imagerie (19) et le deuxième mécanisme d'imagerie (20) sont agencés sur des régions différentes de la même face de la surface de la carte d'imagerie (39),
l'axe optique du premier système optique et l'axe optique du deuxième système optique correspondent à un axe définissant le sens de déplacement de l'appareil insérable dans un corps,
la carte d'imagerie (39) est pliée au niveau de deux parties pliées pour former une section transversale en forme de U et agencée à l'intérieur de l'élément de boîtier extérieur (38), de telle sorte que la face de la surface de la carte d'imagerie (39) sur laquelle le premier mécanisme d'imagerie (19) et le deuxième mécanisme d'imagerie (20) sont agencés est une face d'un côté de région en saillie de la carte d'imagerie (39) en forme de U, et
l'appareil insérable dans un corps comprend en outre des batteries (26b, 26c) connectées en série et agencées dans une région d'espace, qui est formée par une face intérieure d'un côté de partie en renfoncement formé par la forme en U, de telle sorte qu'une cathode d'une batterie et une anode d'une autre batterie sont en contact avec les électrodes, respectivement, formées sur la face intérieure du côté de partie en renfoncement de la carte d'imagerie (39).

2. Appareil (2) insérable dans un corps selon la revendication 1, comprenant en outre :
un générateur de données (27) qui est agencé sur une face de la surface de la carte d'imagerie (39), la face de la surface étant la face de la surface sur laquelle le premier mécanisme d'imagerie (19) et le deuxième mécanisme d'imagerie (20) sont agencés, et est adapté à générer des données d'image sur la base de signaux électriques délivrés en sortie du premier transducteur photoélectrique (19a) et du deuxième transducteur photoélectrique (20a).

3. Appareil (2) insérable dans un corps selon la revendication 2, dans lequel le premier transducteur photoélectrique (19a) et le générateur de données (27) sont électriquement connectés l'un à l'autre par l'intermédiaire d'une configuration de câblage (29a) formée sur la face de la surface de la carte d'imagerie (39) sur laquelle le premier mécanisme d'imagerie (19) et le deuxième mécanisme d'imagerie (20) sont agencés, et
le deuxième transducteur photoélectrique (20a) et le générateur de données (27) sont électriquement connectés l'un à l'autre par l'intermédiaire d'une configuration de câblage (29a) formée sur la face de la surface de la carte d'imagerie (39) sur laquelle le premier mécanisme d'imagerie (19) et le deuxième mécanisme d'imagerie (20) sont agencés.

4. Appareil (2) insérable dans un corps selon la revendication 2, comprenant en outre
une unité de transmission (40) qui est agencée sur la face de la surface de la carte d'imagerie (39) sur laquelle le premier mécanisme d'imagerie (19) et le deuxième mécanisme d'imagerie (20) sont agencés, et est adaptée à transmettre des signaux radio incluant les données d'image générées par le générateur de données (27).
